# EUROPEAN PATENT APPLICATION

(11) **EP 2 062 531 A1**
(43) Date of publication of application: **27.05.2009**
(21) Application number: 07396007.2
(22) Date of filing: 26.11.2007
(51) Int. Cl.: A61B 5/08, A61M 16/04, A61M 16/08

(54) **Multiple function airway adapter**

(71) Applicant: GE Healthcare Finland Oy, 00510 Helsinki (FI)
(72) Inventor: Karlsson, Kai, 00660 Helsinki (FI); Haveri, Heikki, 03150 Huhmari (FI)
(74) Representative: Kanerva, Arto

(57) **Abstract**

An airway sensor includes a gas analyzing apparatus (17) for analyzing at least one respiratory gas and a connecting unit (32) for communication with a remotely located host device (31). The gas analyzing apparatus (17) is connected to a sensor electronics (20) and which sensor electronics is also common for flow measuring device (18) for measuring respiratory gas flow and pressure measuring device (19) for measuring respiratory airway pressure when connected to said sensor electronics. (Fig. 3)

## Description

### FIELD OF THE INVENTION

This disclosure relates generally to an airway sensor.

### BACKGROUND OF THE INVENTION

During anesthesia or in critical care, patients are often mechanically ventilated instead of breathing spontaneously. The patient is connected to the patient circuit of the ventilator or anesthesia machine by intubation: inserting an endotracheal tube to the trachea so that gas can flow trough it into and out of the lung. The breathing circuit typically consists of the endotracheal tube, a Y-piece where the inspiratory and expiratory tubes from the ventilator come together, as well as the ventilator.

It is important that the ventilation meets the patient's needs for correct exchange and administration of oxygen (O₂), carbon dioxide (CO₂), nitrous oxide (N₂O), anesthetic agents and other gases. It is also very important to feed gases at pressures that do not damage the patient's lung. The gases should also be given at suitable respiration rate and tidal volume. Anesthesiologists, respiratory therapists and other qualified clinicians use their professional skills to set the ventilation parameters to optimally meet the needs of the patient. Ventilation is often monitored with a respiratory monitor by measuring essentially in real time the concentrations of oxygen, carbon dioxide, nitrous oxide and anesthetic agent in the breathing gas. Respiratory monitor often also have spirometric measurements for monitoring the pressure and gas flow in the patient circuit. From the gas concentration and gas flow data, it is possible to calculate the patient's consumption of oxygen and production of CO₂.

Recent developments in miniaturization of gas sensors have opened the possibility to measure all respiratory gases such as for example CO₂, N₂O, Oxygen and anesthetic agents with a mainstream gas sensor or combination of sensors, placed on an airway adapter in the patient circuit of a ventilator or an anesthesia machine. The key components of spirometry sensors have also been miniaturized so that it is possible to have the spirometry measurement made as mainstream measurement.

A gas sensor needs real time data about airway pressure to correct for pressure effects to the concentration measurements. With a mainstream gas sensor, this causes extra complications because the sensor is not in pneumatic contact with the gas in the patient circuit. Effects caused by variations in the gas mixture to the flow measurement must be corrected when calculating the airway gas flow. In a respiratory monitor with both gas concentration and spirometry monitoring, the corrections can be performed in the calculation software of the respiratory monitor. So, the respiratory monitor must be able to perform these compensations instead of merely displaying the values received from the sensors. It is also possible that the respiratory monitor receives raw data from the sensors and transmits the necessary data to the sensors so that they then can perform the corrections. This method complicates the communication between the sensors and the respiratory monitor and causes extra software-loads to all three devices.

Mainstream gas sensors transform the measured concentrations to electric signals that are transmitted to the respiratory monitor. This is usually done with a cable that also feeds the operating power from the respiratory monitor to the sensor.

A spirometry sensor converts the airway flow and airway pressure to electric signals that are connected to the respiratory monitor with a cable that also feeds the operating power to the sensor. It is also possible that the measurement is made using a spirometry adapter on the airway generating pressure signals that are conducted to the spirometry unit in the respiratory monitor. Two tubes or one double lumen tube is needed for the pneumatic connection because two signals must be transmitted. Combinations of electric and pneumatic signals can naturally also be used. Pneumatic tubes between spirometry adapter in the patient airway and the respiratory monitor are always a risk, because medical personnel may step on these tubes which may disturb measurement results or even break sensitive components in the spirometry sensor. Both tubes and cables lying on a floor are a security risk for personnel moving around a patient. Also the more cables and their couplings on a monitor the more unreliable the measuring system is. Analog signals sent from sensors to respiratory monitor require thick cables which are not flexible enough and therefore are not convenient.

Further two separate sensors for gas concentrations and spirometry which are at a distance from the respiratory monitor has several drawbacks. One is that at least two cables or one cable plus a tube are needed for the connections between the sensors and the respiratory monitor. From usability point of view, it is very desirable to minimize the number of cables and tubings between the patient and the patient monitor. From reliability point of view, all extra cables, tubes and connectors should be eliminated because connectors and cables are among the most unreliable electric components. Also a drawback is that both sensors must have their own circuitry for generating and regulating their operating voltages, conditioning the analog signals, converting them to digital format, calculating the desired results and communicating them to the respiratory monitor. This tends to increase the size, weight and price of the sensors. In the clinical setting, it is very important to minimize the size and weight of mainstream sensors that are used close to the patient. Especially in neonatal monitoring, it is of utmost importance to minimize the size and weight of mainstream sensors because too heavy sensors may severely limit the movements of the patient and may even cause a risk of the patient falling down from the bed or incubator, drawn by heavy sensors and their cables. Further drawback is that both sensors must have their own softwares for controlling the operation of the sensor, collecting the necessary raw data, calculating the desired parameter values and communicating them to the host monitor. Because of the needs for pressure and gas compensations in the gas and flow sensors, the two sensors must communicate with each other or the respiratory monitor has to serve for data exchange between the two sensors. This causes many complications to the designs of the softwares of all the three devices.

### BRIEF DESCRIPTION OF THE INVENTION

The above-mentioned shortcomings, disadvantages and problems are addressed herein which will be understood by reading and understanding the following specification.

In an embodiment an airway sensor includes a gas analyzing apparatus for analyzing at least one respiratory gas and a connecting unit for communication with a remotely located host device. The gas analyzing apparatus connected to a sensor electronics and which sensor electronics is also common for flow measuring device for measuring respiratory gas flow and pressure measuring device for measuring respiratory airway pressure when connected to said sensor electronics.

In another embodiment an airway sensor includes a sensor electronics, a gas analyzing apparatus connected to the sensor electronics, a flow measuring device connectable to the sensor electronics and a pressure measuring device connectable to the sensor electronics. The sensor electronics is adapted to collect data from one or more of the gas analyzing apparatus, the flow measuring device, and the pressuring measuring device and to calculate one or more parameter value based on the collected data and to selectively transmit the parameter value to a host device via a single connecting unit.

In yet another embodiment, an airway sensor includes a gas analyzing apparatus, a flow measuring device coupled with the gas analyzing apparatus and a pressure measuring device coupled with the gas analyzing apparatus and the flow measuring device. The gas analyzing apparatus, the flow measuring device, and the pressuring measuring device are operatively connectable to a remotely located host device with a single connecting unit.

Various other features, objects, and advantages of the invention will be made apparent to those skilled in art from the accompanying drawings and detailed description thereof.

### BRIEF DESCRIPTION OF THE DRAWINGS

Figure 1 is a general view of an apparatus known in prior art;

Figure 2 is a general view of another apparatus know in prior art;

Figure 3 is a general view of the apparatus of an embodiment in an operating environment;

Figure 4 is a detailed view of one embodiment of the apparatus shown in Figure 3; and

Figure 5 is a detailed view of another embodiment of the apparatus shown in Figure 3.

### DETAILED DESCRIPTION OF THE INVENTION

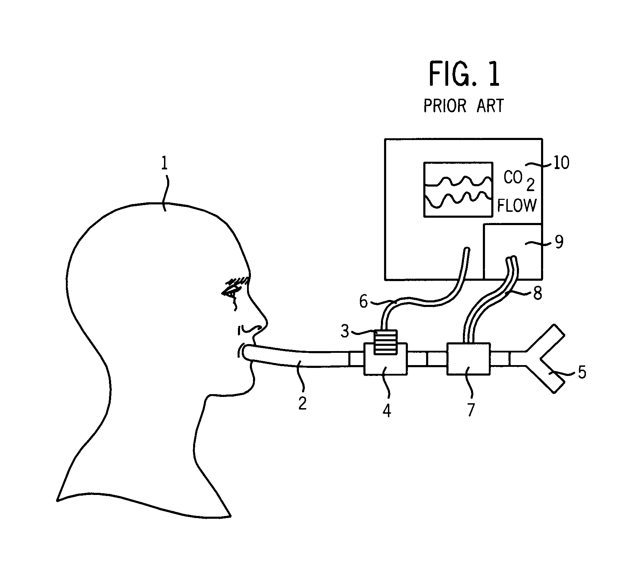

Fig.1 shows a typical known arrangement where the respiratory monitor has both gas concentration measurements and spirometry. An endotracheal tube 2 is placed into a trachea of a patient 1. A gas sensor 3 is placed on an airway gas adapter 4 between the endotracheal tube 2 and a Y-piece 5, so that both inspired and expired gases flow trough it. The gas sensor 3 is electrically connected to the respiratory monitor 10 by cable 6. A spirometry adapter 7 is next to the airway gas adapter 4 between the endotracheal tube 2 and the Y-piece 5. A spirometry tube 8 conducts pressure signals from the spirometry adapter 7 to the spirometry unit 9 in the respiratory monitor 10.

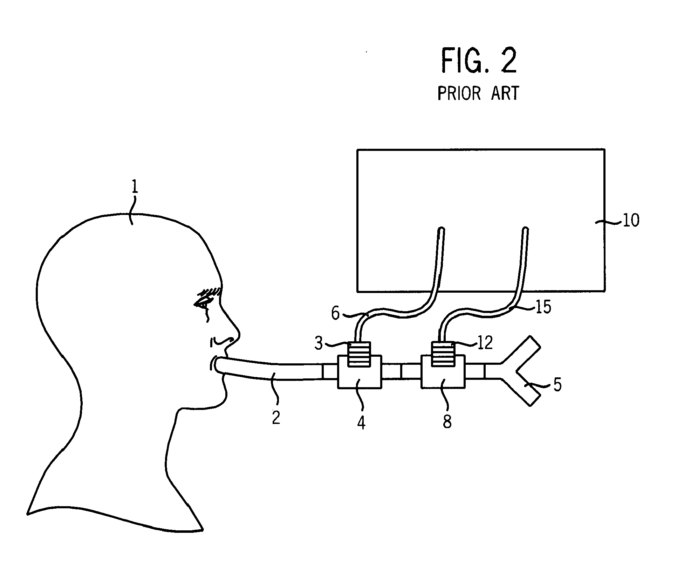

Fig.2 shows a known arrangement where the gas sensor (3) is connected to the respiratory monitor 10 with cable 6 and the spirometry sensor 12 is placed directly on the spirometry adapter 8 on the airway and connected to the respiratory monitor 10 with cable 15. In this case, the respiratory monitor 14 does not contain a spirometry unit.

In the arrangement of Fig.1, it is necessary to have a cable and a double lumen tube between the patient site and the respiratory monitor 10. It is also necessary to have the spirometry unit 9 built in the respiratory monitor 10. In the arrangement of Fig.2, there are two electric cables between the patient site and the respiratory monitor. Thus, in both arrangements, a single electric cable is not sufficient for interfacing the sensors with the respiratory monitor. This is a major drawback, because from usability and reliability points of view it is highly desirable to minimize the number of cables and tubes in patient monitoring systems.

In both arrangements, the respiratory monitor 10 has to communicate the measured airway pressure to the gas sensor for pressure corrections, or alternatively the pressure corrections must be made by the respiratory monitor 10. In both arrangements, the respiratory monitor 10 has to communicate the measured gas concentrations to the spirometry sensor 12 for the necessary corrections to the airway flow, or alternatively the corrections must be made by the respiratory monitor 10.

In both arrangements, it is naturally possible that the respiratory monitor 10 makes the necessary compensations for airway pressure effects to the gas concentrations and corrections for gas concentrations to the airway flow. This is possible when the necessary calculations can be programmed into the respiratory monitor provided that it has sufficient calculating power.

It is possible to use any combinations of electric and pneumatic connections between the spirometry adapter, the spirometry sensor or spirometry unit and the respiratory monitor.

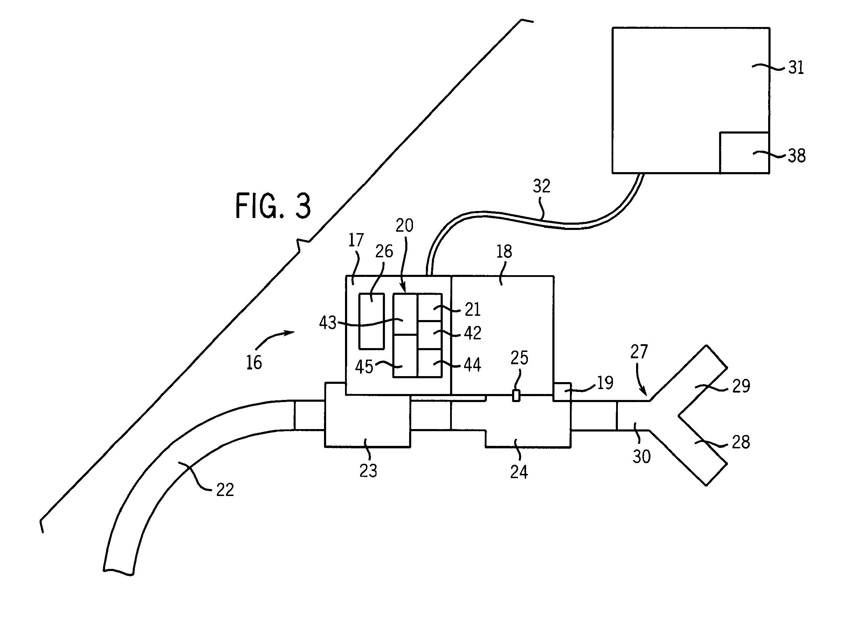

Figure 3 discloses an embodiment of an invention which is a novel airway sensor 16 comprising at least gas analyzing apparatus 17. The gas analyzing apparatus 17 can be connected with flow measuring device 18 and pressure measuring device 19 or there is an option to later connect gas analyzing apparatus 17, flow measuring device 18 and pressure measuring device 19 together. Other component present in the airway sensor 16 is a sensor electronics 20 with a sensor software program. The sensor electronics includes a voltage conversion unit 21, a data processing unit 42, signal conditioning circuitry 43, an analog to digital converter 44 as well as a data interface circuitry 45 .
The voltage conversion unit 21 converts all operating voltages that are needed in the airway sensor and distributes them within the airway sensor 16. The signal conditioning circuits 43 perform the necessary conversions, amplifications and filtrations of raw signals so that they can be converted to digital format with good resolution and signal to noise ratio. The analog to digital converter 44 converts the conditioned signal voltages to digital format as commanded by the data processing unit 42. The data interface circuitry 45 transmits data to the host device 31 and may receive data from the host device 31.
The operation of the data processing unit 42 and the data interface circuitry 45 is controlled by the sensor software program that is stored in the data processing unit 42. The software program performs the collection of the necessary raw data, the calculations needed for gas analysis and flow and pressure measurements and also takes care of the data communication with the host device 31. The common software performs all the necessary corrections to the gas analysis and flow measurement in the correct manner because all necessary data is readily available for it. From the software simplicity point of view it is very good to have only one interface to the host device 31, instead of separate interfaces for gas analyzing apparatus and flow measuring device. Further a voltage conversion unit 21 is needed in case it is necessary to convert voltages and distribute operating voltages within the airway sensor. The airway sensor 16 is connected to a patient airway tube 22, such as an endotracheal tube, by means of an airway gas adapter 23 and a flow measuring adapter 24. Also a pneumatic connection through pressure port 25 is needed for the pressure measuring device 19. Instead of using the patient airway tube 22 it is possible to set a gas tight mask on the patient's face so that the mask covers the patient's mouth and nose and respiratory gases can flow through the mask to and from the patient's airway.

The gas analyzing apparatus 17 having a gas sensor 26 is able to analyze respiratory gas or gases, which analysis may include both qualitative and quantitative analysis or only one of them. The gas sensor 26 includes components that generate the raw signals for the calculations of the concentrations of one or more gases. Many different operating principles of such gas sensor are well known: the gas sensor may measure the infrared absorptions of gases or paramagnetic properties of gases, electrochemical reactions in presence of gases, changes in mechanical, acoustic or thermal properties of gases or other phenomena where the composition of the airway gas mixture can be measured. Embodiments are not related to any particular operating principles of airway gas adapter 23 or flow measuring adapter 24 .The gas sensor 26, the sensor electronics 20 and the voltage conversion unit 21 are advantageously integrated into gas analyzing apparatus 17. The flow measuring device 18 is advantageously detachably connected to the gas sensor 26. The pressure measuring device 19 can be part of the flow measuring device 18.

The airway gas adapter 23 and the flow measuring adapter 24 are placed in the airway between the patient airway tube 22 and a breathing connector 27, such as Y-piece, having three branches, first branch 28 for exhaled gases of a subject, second branch 29 for inhaled gases and third branch 30 for both inhaled and exhaled breathing gases. Both inspired and expired gas flows trough the adapters 23, 24. In case a gas tight mask on the patient's face is used replacing the patient airway tube 22, the airway sensor 16 is placed between the breathing connector 27 and the mask.

The airway gas adapter 23 may include a window for passing radiation, such as infrared energy, emitted by the emitter of the gas sensor 26 to a breathing gas flowing through a channel inside the gas adapter and through another window to the detector of the gas sensor 26.

The flow measuring adapter 24 can be for example a differential pressure adapter or a hot wire anemometer adapter. The differential pressure adapter comprises a flow constricting device inside a channel along which breathing gases are flowing with at least one pressure measuring port on both sides of the flow constricting device and which ports are connected to the flow measuring device 18 sensing the pressure difference of these ports. The flow measuring device18 includes components that generate raw signals for the calculations of airway gas flow. The raw signal may be obtained for example by measuring by a differential pressure sensor the pressure drop that is generated across the constricting element in the flow measuring adapter. The flow signal may also be obtained anemometrically by placing a heated component in to the gas flow in the flow measuring adapters, and measuring the temperature of the heated component, or the electric current that is needed to keep the temperature of the heated component constant. Other measuring methods can also be used.

The pressure measuring device 19 may be in pneumatic connection by means of only one pressure port 25 to the patient airway where respiratory gases are flowing along the patient airway tube 22. This connection can be arranged directly to the patient airway tube 22 or this pressure port 25 is connected through the flow measuring device 18 to the patient airway tube 22 for measuring a pressure in a patient airway. It is typical that the flow measuring adapter 24 having those two ports for pressure difference measurements is able to replace any direct pressure port to the patient airway tube when one of pressure difference measurement ports is connected to both flow measuring device 18 and pressure measuring device 19, but the main thing is that the pressure measuring device 19 is able to sense the pressure of the respiratory air flow. The pressure measuring device includes components that generate raw signals for the calculations of airway pressure.

In the embodiment shown in figure 3, the airway sensor 16 is connected with a host device 31 by connecting unit 32, which can be a cable or wireless connection.

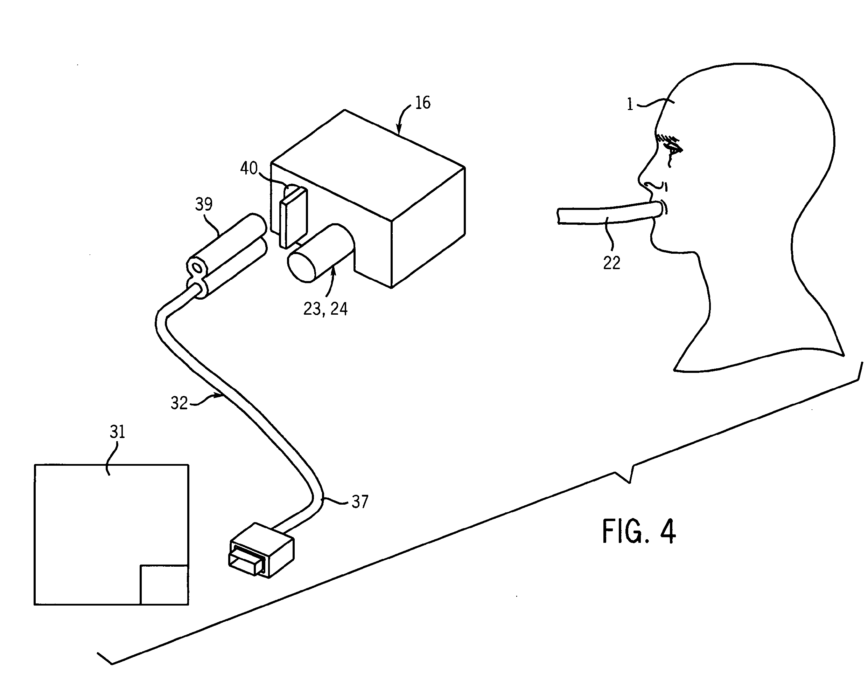

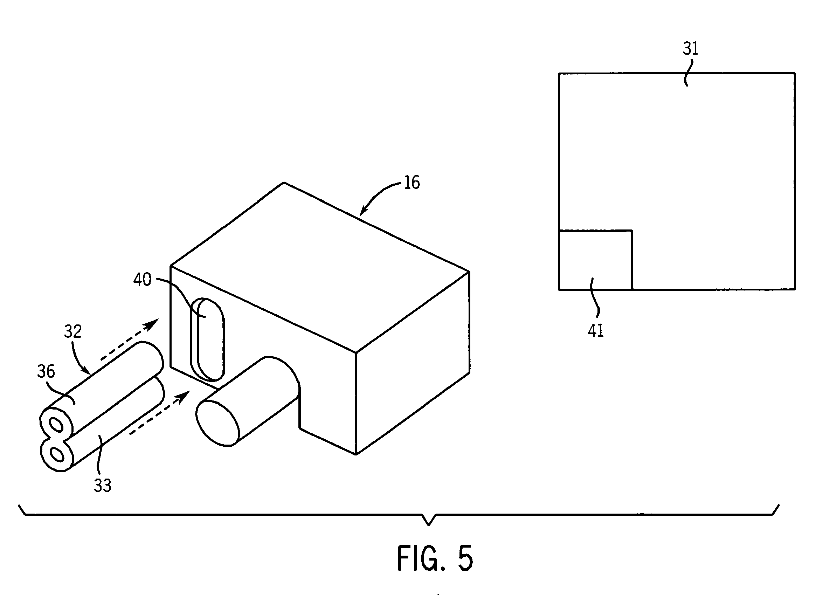

According to an embodiment shown in figure 4 the airway sensor 16 has a separate connector 40 for the connecting unit 32. If the connection is made with a cable, then a connector 39 on the airway sensor end of the cable is connected to a mating connector 40 of the airway sensor 16. It is possible to arrange the communication between the host device 31 and the airway sensor 16 so that all data is transmitted wirelessly e.g. by radio frequency link. If the connection is wireless as shown in figure 5, the connecting unit 32 includes an energy store 33, which may be like a battery or accumulator, that stores and supplies the operating power to the airway sensor 16, and a wireless communication circuitry 36 for wireless communication between the airway sensor 16 and the host device 31 and which host device 31 includes wireless interface circuitry 41 for communication with airway sensor 16.

The advantage is that only one interface between the host device 31 and the airway sensor 16 is needed. Thus, if a cable is used for interfacing, only one cable between the host device 31 and the airway senor 16 is needed. The host device 31 can be e.g. a monitor, anesthesia machine, ventilator or even personal computer. The remotely located host device 31 receives by means of the connecting unit 32 used for communication one or more parameter value calculated based on the collected data by the sensor electronics 20. In some embodiments analysis and measurement results from the airway sensor are shown on the display 38 of the host device 31 or which display can be a separate device but controlled by the host device. Also these analysis and measurement results can be only used by the host device 31 for other purposes and not displayed as such. Because all necessary corrections to the gas concentrations and airway flow are performed in the airway sensor 16, only a suitable interface, e.g. USB, and the basic functionality for displaying the results is needed in the host device 31. The host device 31 feeds necessary operating voltages to the voltage conversion unit 21 in the gas analyzing apparatus 17, if the connecting unit 32 is the cable, and the voltage conversion unit feeds the operating power to the flow measuring device 18.

The flow measuring device 18 can be connected to the gas analyzing apparatus 17 by galvanic contacts. It is possible to avoid galvanic contacts for signal transmission by using wireless communication between the flow measuring device 18 and the gas analyzing apparatus 17. This can be accomplished by opto-electronic links or by radio frequency communication.

It is possible to avoid galvanic contacts for feeding the operating power to the flow measuring device 18 using a transformer whose primary circuit is in the gas analyzing apparatus 17 and secondary circuit is integrated with the flow measuring device 18. In this case, the voltage conversion unit 21 is divided so that its primary part is in the gas analyzing apparatus 17 and its secondary part is with the flow measuring device 18.

The airway sensor 16 has several substantial advantages. An advantage is that only one connecting unit 32 is needed between the airway sensor 16 and the host device 31, even if the gas analyzing apparatus 17, the flow measuring device 18 and the pressure measuring device 18 are in use. This is in very good accordance with the user's requirements to minimize the number of cables and tubings between the patient and the host device 31. Also an advantage is that all conversions of operating voltages, all analog to digital conversions and all calculations are performed by common data processing unit in the sensor electronics 20. This makes the airway sensor 16 smaller and lighter than solutions with two separate sensors. This is well in accordance with the user's need for small and light weight sensors, especially for neonatal applications. Further advantage is that only one software is needed for the airway sensor 16, calculating all the parameter values and performing all the necessary corrections for gas concentrations and airway pressure. The needs for data communications between two sensors are totally eliminated. This creates an opportunity to simplify the structure and development of the software, thus minimizing the possibilities to software errors. Thus, more reliable and correct operation of the sensor software can be accomplished in the airway sensor than with two separate sensors.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An airway sensor comprising:
a gas analyzing apparatus (17) (23) for analyzing at least one respiratory gas;
a connecting unit (32) for communication with a remotely located host device (31),
**characterized in that** said gas analyzing apparatus (17) connected to a sensor electronics (20) and which sensor electronics is also common for flow measuring device (18) for measuring respiratory gas flow and pressure measuring device (19) for measuring respiratory airway pressure when connected to said sensor electronics.

2. An airway sensor according to claim 1, **characterized in that** said sensor electronics (20) comprise a voltage conversion unit (21), a data processing unit (42), a signal conditioning circuitry (43), an analog to digital converter (44) and a data interface circuitry (45).

3. An airway sensor according to claim 1 or 2, **characterized in that** said sensor electronics (20) includes a software program performing a collection of a necessary raw data from said gas analyzing apparatus (17), flow measuring device (18) and pressure measuring device (19), calculations needed for gas analysis and flow and pressure measurements and also takes care of the data communication with said host device.

4. An airway sensor according to claim 1, **characterized in that** said connecting unit (32) is a wireless connection between said airway sensor (16) and said host device (31).

5. An airway sensor according to claim 4, **characterized in that** said connection unit (32) being the wireless connection, said connection unit comprising:
a wireless communication circuitry (36) for communication with said host device (31) and which wireless communication circuitry is connectable to a mating connector (40) of said airway sensor (16);
an energy store (33) for the function of said airway sensor (16), and which energy store is connectable to said mating connector (40) of said airway sensor (16);
a wireless interface circuitry (41) in said host device (31) for communication with said airway sensor (16).

6. An airway sensor according to claim 1, **characterized in that** said connecting unit (32) comprises a cable (37) and a connector (39) connectable to a mating connector (40) for transferring analysis and measurement results from said airway sensor (16) to said host device (31) and feeding operating voltages from said host device to said airway sensor.

7. An airway sensor according to claim 1, **characterized in that** said gas analyzing apparatus (17) mounted on an airway gas adapter (23) is adapted to make a quantitative analysis of at least one respiratory gas like CO₂, N₂O, Oxygen or anesthetic agent.

8. An airway sensor according to claim 1, **characterized in that** said gas analyzing apparatus (17) mounted on an airway gas adapter (23) is adapted to make a qualitative analysis of at least one respiratory gas like CO₂, N₂O, Oxygen or anesthetic agent.

9. An airway sensor according to claim 1, **characterized in that** a flow measuring device (18) is adapted to be mounted on a flow measuring adapter (24) and a pressure measuring device (19) is adapted to be pneumatically connected by means of a pressure port (25) to respiratory gas flow;

10. An airway sensor according to claim 9, **characterized in that** said flow measuring adapter (24) is a differential pressure adapter.

11. An airway sensor according to claim 9, **characterized in that** said flow measuring adapter (24) is a hot wire anemometer.

12. An airway sensor according to claim 9, **characterized in that** said pressure port (25) for said pressure measuring device (19) is pneumatically connected to the respiratory gas flow through said flow measuring adapter (24).

13. An airway sensor according to claim 1, **characterized in that** said host device (31) is one of a patient monitor, anesthesia machine, ventilator or personal computer.

14. An airway sensor according to claim 1, 7, 8 or 9, **characterized in that** said airway sensor (16) is a single module and which is detachable from adapters (23,24).

15. An airway sensor according to claim 1, **characterized in that** said flow measuring device (18) is detachable from said airway sensor (16).

16. An airway sensor according to claim 1, **characterized in that** a connecting unit (32) for communication with a remotely located host device (31) is adapted to transfer results including parameter values calculated based on the collected data by said sensor electronics (20).

17. An airway sensor according to claim 16, **characterized in that** said host device is configured to show received results on the display (38).

18. An airway sensor according to claim 7, 8 or 9, **characterized in that** said airway gas adapter (23) through which respiratory gas flow and said flow measuring adapter (24) through which respiratory gas flow and a pressure port (25) in pneumatic connection with respiratory gas flow are attachable between said patient airway tube (22) and a breathing connector (27) having three branches, a first branch (28) for exhaled gases, a second branch (29) for inhaled gases and a third branch (30) for both inhaled and exhaled gases.
